# EUROPEAN PATENT APPLICATION

(11) **EP 2 277 866 A1**
(43) Date of publication of application: **26.01.2011**
(21) Application number: 09163351.1
(22) Date of filing: 22.06.2009
(51) Int. Cl.: C07D 231/56

(54) **Process for preparing telmisartan**

(71) Applicant: INKE, S.A., 08755 Castellbisbal (ES)
(72) Inventor: Santosh, Singh, Mahape, Navi Mumbai - 400701 (IN); Anil, Joshi, Mahape, Navi Mumbai - 400701 (IN); Ravindranath, Newadkar, Mahape, Navi Mumbai - 400701 (IN)
(74) Representative: Ponti Sales, Adelaida

(57) **Abstract**

The present invention refers to an improved process for the preparation of telmisartan or a pharmaceutical acceptable salt thereof through hydrolysis compounds of formula general I wherein R is selected from *tert*-butyl ester (Ia) or from nitrile (Ib) or salts thereof, using the same reaction conditions and reagents. Preferably, the invention discloses the preparation of telmisartan through hydrolysis of novel organic salts of compounds of formula I, particularly wherein R is *tert*-butyl ester (Ia). The invention also relates to a process for the preparation of these novel organic salts, in particular wherein R is *tert*-butyl ester(Ia) and its use in the preparation of telmisartan.

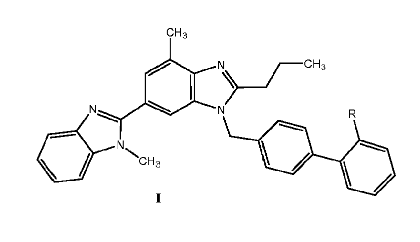

## Description

### FIELD OF THE INVENTION

The present invention refers to an improved process for the preparation of telmisartan or a pharmaceutical acceptable salt thereof through hydrolysis of compounds of formula general I, wherein R is selected from *tert*-butyl ester (Ia) or from nitrile (Ib), or salts thereof, using the same reaction conditions and reagents. Preferably, the invention discloses the preparation of telmisartan through hydrolysis of novel organic salts of compounds of formula general I, particularly wherein R is *tert-*butyl ester (Ia). The invention also relates to a process for the preparation of these novel organic salts, in particular wherein R is *tert*-butyl ester (Ia), and its use in the preparation of telmisartan.

### BACKGROUND OF THE INVENTION

Telmisartan is an angiotensin II receptor antagonist developed for the treatment of hypertension. Its chemical name is 4'-[2-n-propyl-4-ethyl-6-(1-methylimidazol-2-yl)benzimidazol-1-ylmethyl]-biphenyl-2-carboxylic acid having the following structure:

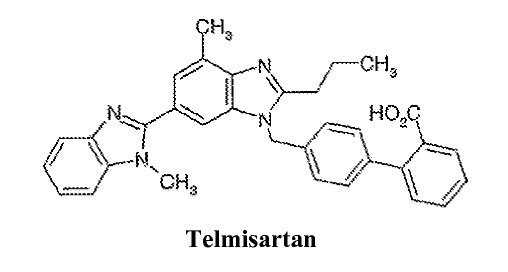

Telmisartan is generally supplied in the free acid form. It is characterised by its very poor solubility in aqueous systems at the physiological pH range of the gastrointestinal tract of between pH 1 to 7.

Telmisartan was first disclosed by the European patent EP 0 502 314 B1 to Boehringer Ingelheim. This patent discloses the preparation of telmisartan by reacting 2-n-propyl-4-methyl-6-(1'-methylbenzimidazol-2'-yl)benzimidazole (II) with *tert-*butyl 4'-bromomethylbiphenyl-2-carboxylate (III) and subsequently hydrolysis, thermolysis or hydrogenolysis of compound Ia. The J. Med. Chem. 1993, 36, 4040-4051 describes the preparation of telmisartan by hydrolysis of the *tert*-butyl ester functionality using trifluoroacetic acid (TFA) and dichloromethane as reaction solvent. Telmisartan is obtained after chromatographic separation as a white solid (Scheme 1).

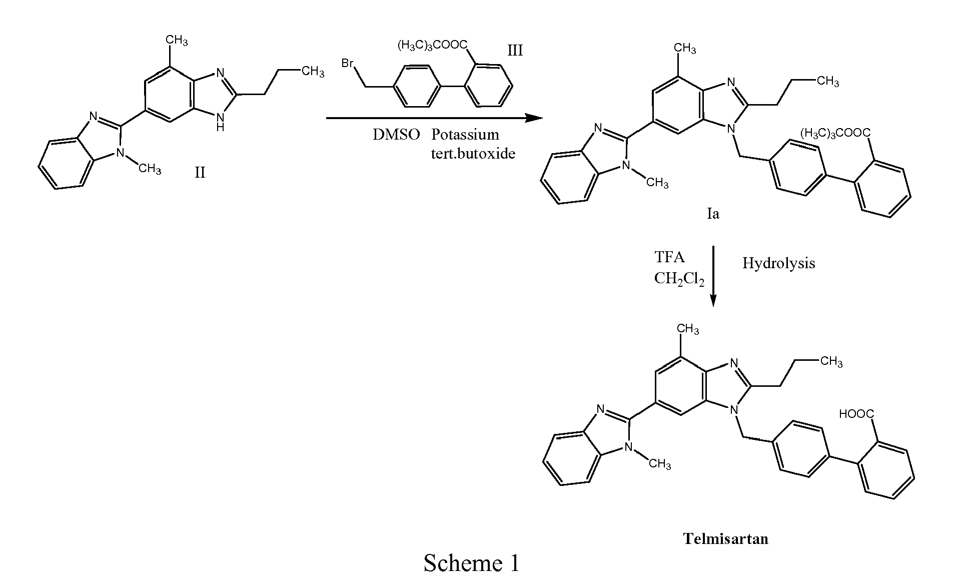

The process of Scheme 1 has been found to have several disadvantages in the industrial manufacture of a pharmaceutical like lengthy reaction times, low purity of the resultant compound and the usage of hazardous chemicals like trifluoroacetic acid.

Moreover, as described in the J. Med. Chem. 1993, 36, 4040-4051, column chromatography is required in order to obtain pure telmisartan. This process is time consuming and consequently, not as economical as the yield obtained is low and not usable at industrial scale.

EP 1 144 386 B1 discloses polymorphs of telmisartan, particularly polymorphic Form B, polymorphic mixtures and their preparation. The disclosure refers to the fact that Form A, obtained according to the basic patent, is difficult to filter, requires a very long drying time and exhibits a strong tendency to electrostatic charging.

Chinese patent application CN 1412183 describes an alternative process for the preparation of telmisartan which includes reacting 2-n-propyl-4-methyl-6-(1'-methyl-benzoimidazol-2'-yl)benzimidazole (II) with 4-bromomethyl-2'-cyanobiphenyl (IV), followed by hydrolysis of the cyano group (compound Ib) to afford telmisartan. In Example 3 of this application the hydrolysis of the cyano group of compound Ib is performed with a two-fold volumetric excess of concentrated hydrochloric acid (e.g. 300 ml) per 0.05 moles of the cyano-telmisartan derivative at a temperature of 100°C, which is a not environmentally friendly process. Finally, upon crystallization of the crude telmisartan from N,N-dimethylformamide (DMF), a material having a purity of 99.0 % was obtained, which is insufficient for pharmaceutical purposes. This process is depicted in Scheme 2.

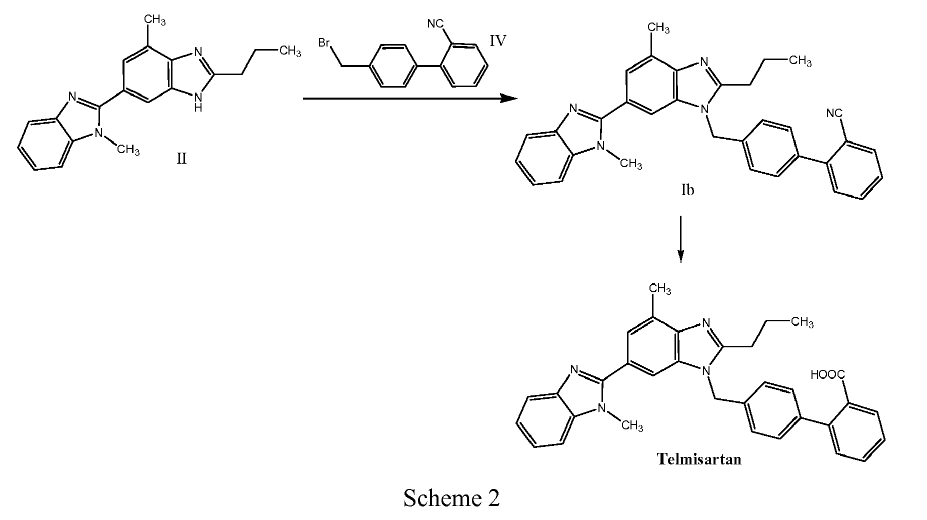

US 7,193,089 B2 also discloses a process for the preparation of telmisartan using 4-bromomethyl-2'-cyanobiphenyl (IV). The product thus obtained yields telmisartan after hydrolysis of the nitrile function into a carboxyle group. This hydrolysis of the nitrile function is, preferably, carried out in a high-boiling solvent system selected from ethylene glycol/water and propylene glycol/water, in the presence of either an acid or a base, potassium hydroxide being particularly suitable, at temperatures between 140°C and 200°C. This reaction is followed by distilling off the solvent and adding concentrated hydrochloric acid to obtain telmisartan hydrochloride, which crystallizes upon cooling. Free telmisartan is obtained after addition at 80-90°C of 4N NaOH. This process is lengthy and the use of high boiling solvents is not desirable when scaling up.

WO 2007/010558 Al to Matrix describes a process for the preparation of telmisartan which involves the use of telmisartan dihydrochloride. The process comprises treating 4-methyl-6-(1-methylbenzimidazol-2-yl)-2-n-propyl-1H-benzimidazole with *tert*-butyl-4'-(bromomethyl)-2-biphenyl-2-carboxylate in the presence of a base in an organic solvent followed by treatment with mineral acid. The resulting acid addition salt of 4'-[4-Methyl-6-(1-methy-1H-benzimidazol-2-yl)-2-n-propyl-1H- benzimidazol-1-yl-methyl]biphenyl-2-carboxylate *tert*-butyl-ester is treated with aqueous hydrochloric acid yielding telmisartan dihydrochloride which is finally converted to telmisartan by using an ammonia solution (Scheme 3).

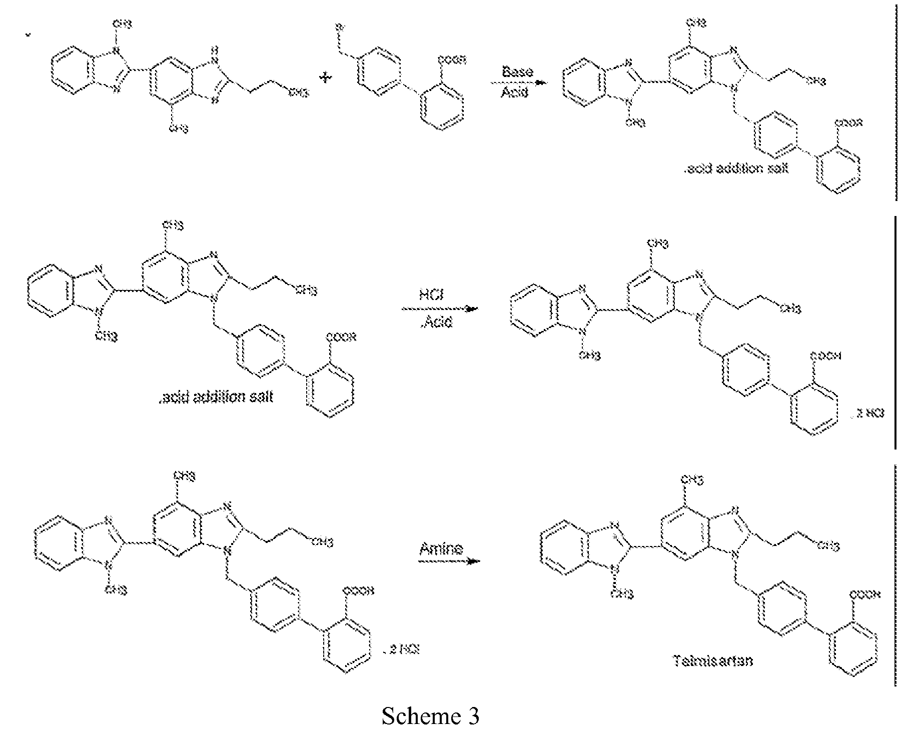

This described process involves the isolation of two salts: the acid addition salt of telmisartan ester and the telmisartan dihydrochloride salt, thus increasing the number of steps to reach the final product and, consequently, its cost. The hydrolysis of the ester is performed by addition of hydrochloric acid which requires acid resistant equipment.

There is a need for processes for the preparation of telmisartan that are environmentally friendly, easy to practice, produce high yields of telmisartan, less costly and that can be adapted to industrial scale.

### BRIEF DESCRIPTION OF THE INVENTION

The present invention is related to an improved process for the preparation of telmisartan or a pharmaceutical acceptable salt thereof through hydrolysis of compounds of formula general I, wherein R is selected from *tert*-butyl ester (Ia) or from nitrile (Ib), or salts thereof, using the same reaction conditions and reagents.

Thus, a first aspect of the invention relies on a process for the preparation of telmisartan or salts thereof which comprises hydrolysis of compounds of general formula I or salts thereof 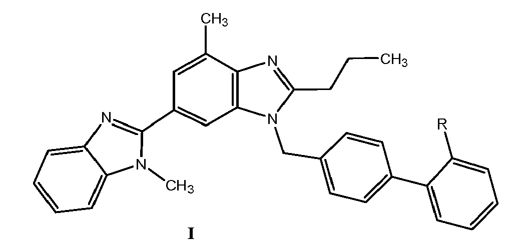 characterized in that the hydrolysis is carried out in a reaction medium comprising an aqueous basic solution and a polar protic solvent and in that R is selected from COOC(CH₃)₃ (Ia) and CN (Ib).

A second aspect of the invention is to provide novel salts of compounds of formula general I, particularly, wherein R is COOC(CH₃)₃ (Ia) and wherein HA is an organic acid:

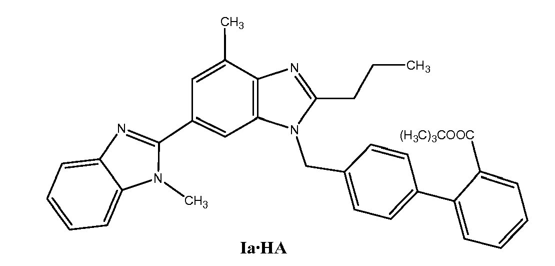

A preferred aspect of the present invention is to provide a process for the preparation of telmisartan or salts thereof which comprises the hydrolysis of salts of the compound of formula Ia·HA, then, the isolation of the obtained crude telmisartan and, optionally, the purification of crude telmisartan.

A third aspect of the invention is to provide a process for the preparation of said novel salts (Ia·HA).

A fourth aspect of the present invention is the use of said salts of formula Ia·HA, wherein HA is an organic acid, for the preparation of telmisartan.

### DETAILED DESCRIPTION OF THE INVENTION

The present invention provides an improved process for obtaining telmisartan through the hydrolysis of either the *tert*-butyl ester derivative (Ia) or the nitrile derivative (Ib) of telmisartan or salts thereof. The process of the invention allows the production of telmisartan with a high purity in a reduced number of reaction steps and it can be applied at an industrial level with low energy and costs.

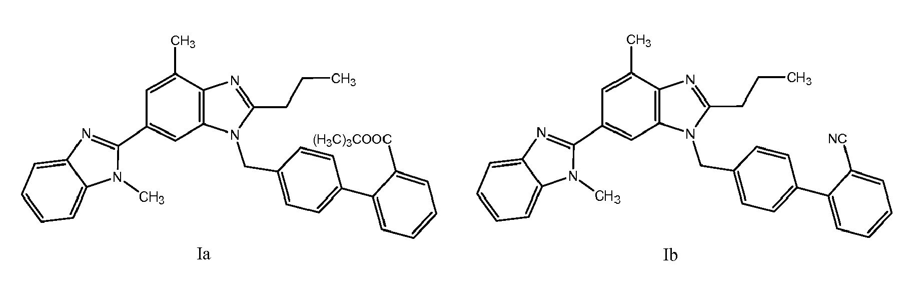

The authors of the invention have surprisingly found that the use of a reaction medium comprising an aqueous basic solutionand a polar protic solvent allows the hydrolysis of both compounds Ia and Ib, or its salts, yielding crude telmisartan in high yields and purity.

As used herein an aqueous basic solution comprises an inorganic base. Preferably, the inorganic base is selected from the group consisting of a metal hydroxide. Preferably, the metal hydroxide is sodium hydroxide, potassium hydroxide, cesium hydroxide, barium hydroxide, magnesium hydroxide or strontium hydroxide. Preferably, the aqueous basic solution is a NaOH solution 60% (w/w).

As used herein, the term "polar protic solvent" refers to a polar solvent that is capable of exchanging protons with the reagents and that contains polarizable proton. Examples of polar protic solvents are butanol, isopropanol (IPA), propanol, ethanol, methanol, water and mixtures thereof. Preferably, the polar protic solvent is butanol.

The reaction can be performed in a temperature range between 80°C and 125°C, preferably, between 115°C and 120°C.

During isolation of the product obtained after the hydrolysis, pH is adjusted to 4,5-5,0 using acetic acid, dilute HCl or IPA·HCl.

In a preferred embodiment, the reaction is carried out by the use of phase transfer catalyst such as tetrabutyl ammonium hydrogen sulfate, tetrabutyl ammonium bromide and 18-crown-6-ether, preferably, tetrabutyl ammonium hydrogen sulfate. A 0,5-1,5%, preferably, 1% catalyst loading is used in the reaction.

Surprisingly, the authors have discovered that, good results can also be obtained when the hydrolysis reaction is performed on compound Ia or salts thereof, without the presence of the phase transfer catalyst. Moreover, when compound Ia or salts thereof are the starting material, the reaction time is shorter.

Advantageously, novel organic salts of formula Ia·HA according to one of the aspects of the present invention are easy to isolate, manipulate and purify. Then, it would be preferably to use these salts as starting materials in the preparation of telmisartan with high yield and purity. Optionally, it is possible to obtain telmisartan without the isolation of telmisartan ester intermediate, Ia.

The hydrolysis of novel organic salts of formula Ia·HA in a reaction medium comprising an aqueous NaOH solution 60% (w/w) and butanol yields telmisartan within 3-4 hours at 115°-120°C with good yield and purity.

Accordingly, a second aspect of the present invention relies on novel salts of the compound of formula Ia·HA: 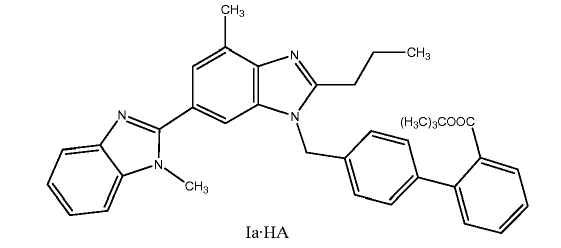 wherein HA is an organic acid.

In a preferred embodiment, said organic acid is selected from fumaric acid, oxalic acid, succinic acid, citric acid, maleic acid, tartaric acid, trifluoroacetic acid, p-toluene sulfonic acid and methane sulfonic acid, preferably the oxalic acid.

The salts of the invention are easily and directly prepared over the product obtained from the condensation between 2-propyl-4-methyl-6-(1'-methylbenzimidazole-2-yl)benzimidazole and *tert*-butyl-4-bromomethylbiphenyl-2-carboxylate and, optionally, without the isolation of compound Ia. Scheme 4 shows the preparation of said salts according to the invention.

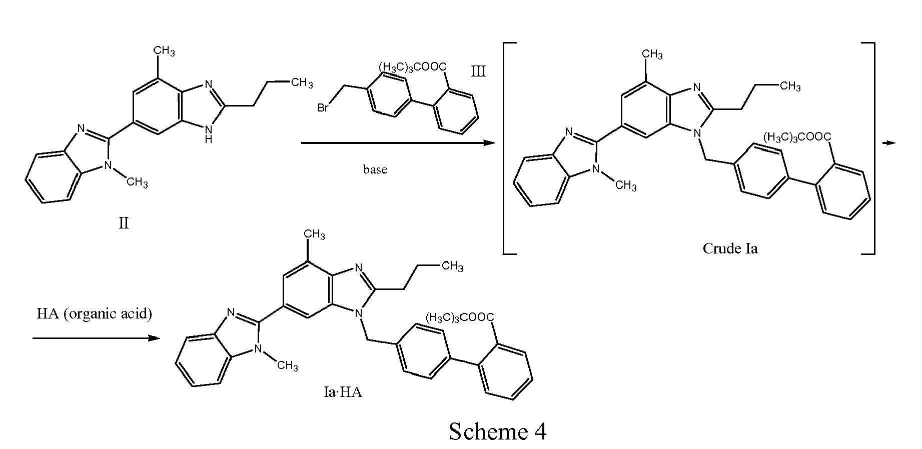

Thus, a third aspect of the invention relies on a process for the preparation of said novel salts of formula la·HA which comprises:
a) Reacting 4-methyl-6-(1-methylbenzimidazol-2-yl)-2-n-propyl-1H-benzimidazole with *tert*-butyl-4'-(bromomethyl)-2-biphenyl-2-carboxylate in the presence of a base in an organic solvent
b) Adding an organic acid (HA)
c) Optionally, purifying the obtained salt of formula Ia·HA.

According to a preferred embodiment the base is selected from potassium *tert-*butoxide, sodium methoxide, sodium ethoxide, potassium hydroxide and the like, preferably potassium *tert*-butoxide.

Step a) of the process may proceed in the presence of a wide variety of organic solvents. Election of the most suitable solvent or mixture of solvents requires only routine experimentation for the skilled person. Preferably, said organic solvent is selected from dimethylsulfoxide, acetone, methanol, dimethylformamide, isopropanol, ethyl acetate, acetonitrile and mixtures thereof. Preferably, the solvent is dimethylsulfoxide.

The product of step a), compound Ia, can be, optionally, isolated and dried before performing the acid addition of step b).

The solvent used in step a) of the process can be the same of the step b) or it can be a mixture of solvents or another different organic solvent. Preferably, said organic solvent is selected from ethyl acetate, n-propyl acetate, methylene dichloride (MDC), isopropyl alcohol, methanol, ethanol and methyl ethyl ketone or mixtures thereof. The organic acid used in step b) is selected from fumaric acid, oxalic acid, succinic acid, citric acid, maleic acid, tartaric acid, trifluoroacetic acid, p-toluene sulfonic acid and methanesulfonic acid, preferably the oxalic acid. The acid can be added directly or dissolved in a suitable organic solvent. Election of the most suitable solvent or mixture of solvents requires only routine experimentation for the skilled person. Preferably, said organic solvent is methanol.

The process may comprise further purification of the salt of formula Ia·HA (step c) to achieve a purer compound. For example, the salt can be purified by suspending or dispersing the crude salt in an organic solvent while heating at reflux. Organic solvents useful for this purification are selected from methyl ethyl ketone, acetone, ethylacetate, isopropylalcohol, isopropylacetate, ethanol and the like. Preferably, methyl ethyl ketone.

A preferred process for the preparation of telmisartan is to carry out the hydrolysis as described above on the salt of the compound of formula Ia·HA, wherein HA is an organic acid, 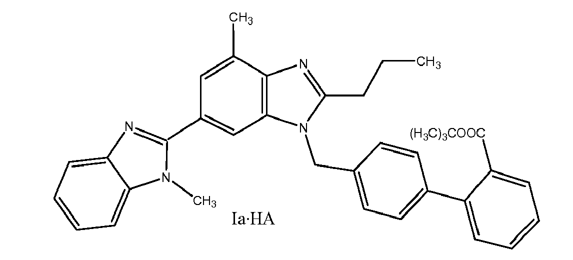 then the isolation of the obtained crude telmisartan and, optionally, the purification of crude telmisartan.

The isolation of the crude telmisartan obtained by hydrolysis of compound of formula Ia·HA requires pH adjustment to 4,5-5,0 using acetic acid, dilute HCl or IPA·HCl, preferably IPA·HCl. Crude telmisartan prepared according to the preferred process of the present invention presents a HPLC purity of at least 99.7%. Additionally, and extra purification may be done.

The authors of the invention have prepared telmisartan of high purity from different organic salts of the compounds of formula general I using fumaric acid, oxalic acid, succinic acid, citric acid, maleic acid, tartaric acid, trifluoroacetic acid, p-toluenesulfonic acid and methanesulfonic acid. Best results are achieved with fumaric and oxalic acids. As it is shown on table 1 below, the highest purity reached was when oxalic acid is used to obtain the corresponding telmisartan butyl ester oxalate.

**Table 1**

| **HA** | **% Purity Salt of Telmisartan ester** | **% Purity crude Telmisartan** | **% Purity pure Telmisartan** |
|---|---|---|---|
| Fumaric | 98,80 | 99,35 | 99,48 |
| Oxalic | 99,70 | 99,74 | 99,83 |

In a fourth aspect, the invention relies on the use of said novel salts of formula Ia·HA, wherein HA is an organic acid, in the preparation of telmisartan.

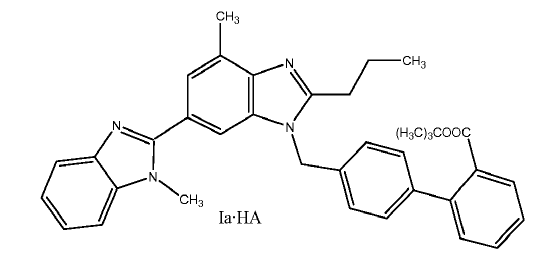

The invention may also comprise further purification of crude telmisartan so as to achieve a highly pure compound. Preferably, telmisartan is subjected to purification by suspending crude telmisartan in ethanol, acetone or methanol and addition of ammonium hydroxide solution or liquid ammonia or methanolic ammonia. The solution is charcoalized, filtered and then adjusted the pH to 4.5 to 5.0 with acetic acid to get purified telmisartan. The authors have found that the use of ethanol, liquid ammonia and glacial acetic acid is suitable for good quality and yield. Preferably, pH adjusting to 4.5-5.0 is suitable for better recovery. Telmisartan purified as described, presents a purity of at least 99.8 % HPLC.

Telmisartan as prepared and isolated by a process according to the present invention comprises polymorphic Form A as defined in EP 1 144 386 B1.

Telmisartan obtained according to the process of the present invention can be milled or micronised to obtain a D₅₀ and D₉₀ particle size of less than about 400 µm, preferably less than about 200 µm, more preferably less than about 150 µm, still more preferably less than about 50 µm and most preferably less than 15 µm. It is noted the notation Dₓ means that X% of the particles in a composition have a diameter less than a specified diameter D. Thus, a D₅₀ of about 400 µm means that 50% of the micronised telmisartan particles have a diameter less than 400 µm.

Particles of this size are obtained by conventional methods, e.g. grinding in an air jet mill, hammer and screen mill, fine impact mill, ball mill or vibrator mill.

Micronisation is preferably effected by known methods using an ultrasonic disintegrator or by stirring a suspension with a high speed agitator.

In the following, the present invention is further illustrated by examples. They should in no case be interpreted as a limitation of the scope of the invention as defined in the claims.

### EXAMPLES

### Example 1. Preparation of the oxalate salt of Telmisartan tert-butyl ester (3)

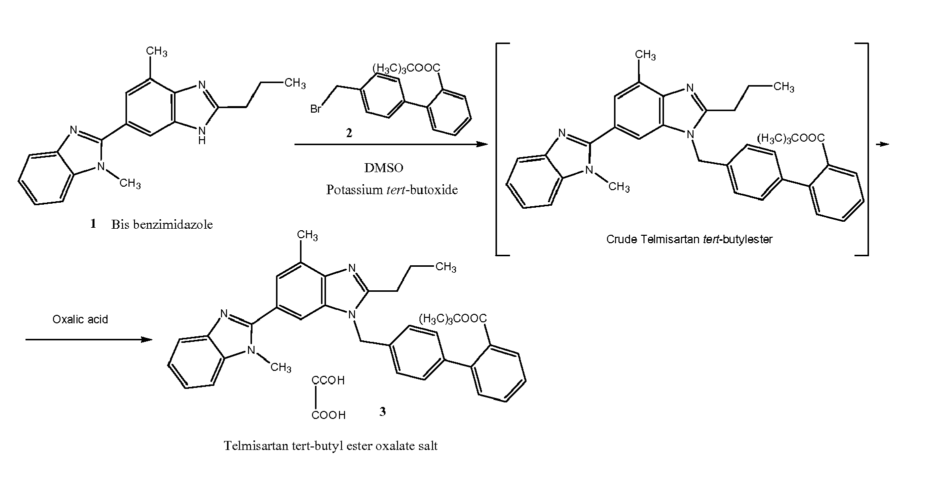

### 1.1. Condensation Step: Formation of crude Telmisartan tert-butylester

10 g (0.0329 mol) of 2-propyl-4-methyl-6-(1'-methylbenzimidazole-2-yl)benzimidazole (1) were dissolved in 60 ml of dimethylsulfoxide (DMSO). The resulting solution was stirred at room temperature (25-30°C) until the obtaining of a clear solution. 3.7 g (0.0329 mol) of Potassium *tert*-butoxide were added to the aforementioned solution and stirred at room temperature (25-30°C) for 30 min. Afterwards, 11.45 g (0.0329 mol) of *tert*-Butyl-4-bromomethylbiphenyl-2-carboxylate (2) was added and the obtained mixture was stirred at room temperature (25-30°C) for 2 h. At this stage, the reaction was quenched with water (400 ml) and 200 ml of ethyl acetate was added. The aqueous layer was separated and extracted again with 100 ml ethyl acetate. The organic layers were combined and washed twice with water (100 ml each). Afterwards, the resulting organic solution was dried with sodium sulphate and filtered. The solvent was partially removed under vacuum.

### 1.2. Oxalate saltformation (3)

The ethyl acetate solution, generated in the above condensation step 1.1., was treated with a solution of 4.56 g (0.0361 mol) of oxalic acid dissolved in hot methanol (6 ml). The resulting mixture was refluxed for 3 h. The reaction mass was cooled down to room temperature (25-30°C), stirred for 3 hours, filtered and the obtained solid was washed with ethyl acetate (10 ml). The resulting wet solid was stirred under reflux with methyl ethyl ketone (150 ml) for 2 hours. The reaction mass was then cooled down to 20°C, stirred for 2 hours, filtered and washed with methyl ethyl ketone (10 ml). The resulting oxalate salt **3** was dried under vacuum at 80°C until constant weight.

| | |
|---|---|
| *Yield:* | *16.2 g* |
| *Yield (molar):* | *75%* |
| *HPLC:* | *99.2%* |
| *M.P.:* | *165-175°C* |

### Example 2. Preparation of the oxalate salt of Telmisartan tert-butyl ester (3)

### 2.1. Condensation Step: Formation of crude Telmisartan tert-butylester

10 g (0.0329 mol) of 2-propyl-4-methyl-6-(1'-methylbenzimidazole-2-yl)benzimidazole **(1)** was dissolved in 60 ml of dimethylsulfoxide (DMSO). The resulting solution was stirred at room temperature (25-30°C). 3.7 g (0.0329 mol) of Potassium *tert*-butoxide was added to the aforementioned solution and stirred at room temperature (25-30°C) for 30 min. Afterwards, 11.45 g (0.0329 mol) of *tert*-Butyl-4-bromomethylbiphenyl-2-carboxylate **(2)** was added and the obtained mixture was stirred at room temperature (25-30°C) for 2-3 h. At this stage, 100 ml of MDC and 400 ml of water were added and the reaction stirred for 30 min. The organic and aqueous layers were separated. The aqueous layer was extracted with MDC. The organic layers were combined and washed twice with water and once with NaCl solution. The resulting organic layer was dried over sodium sulphate and filtered.

### 2.2. Oxalate saltformation (3)

To the above organic solution, 4.56 g (0.0361 mol) of oxalic acid dissolved in hot methanol (6 ml) were added. The resulting mixture was refluxed and the solvent removed partially at 40-45°C under atmospheric pressure. The reaction mass was stirred for 3 h at same temperature. The reaction mass was cooled to 10-15 °C and stirred for 3 h. Afterwards, the suspension was filtered and the obtained solid was washed with methyl ethyl ketone (20 ml). The resulting wet solid was stirred with methyl ethyl ketone (100 ml) under reflux for 2 hours. The reaction mass was cooled down to 15-20°C and stirred for 2 h. The reaction mass was filtered and the solid was washed with methyl ethyl ketone. The resulting oxalate salt **3** was dried under vacuum at 80-82°C until constant weight.

| | |
|---|---|
| *Yield:* | *16. 0g* |
| *Yield (molar):* | *73.9%* |
| *HPLC:* | *99. 6%* |

### Example 3. Preparation of the oxalate salt of Telmisartan tert-butyl ester (3)

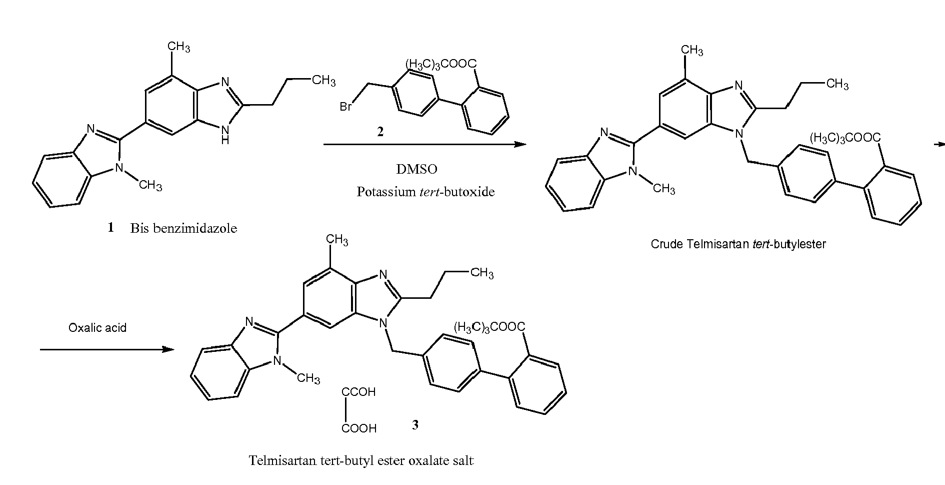

### 3.1. Condensation Step: Formation of crude Telmisartan tert-butylester

10 g (0.0329 mol) of 2-propyl-4-methyl-6-(1'-methylbenzimidazole-2-yl)benzimidazole **(1)** was dissolved in 60 ml of dimethylsulfoxide (DMSO). 3.7 g (0.0329 mol) of Potassium *tert*-butoxide was added to the aforementioned solution and stirred at room temperature (25-30°C) for 30 min. Afterwards, 11.45 g (0.0329 mol) of *tert*-Butyl-4-bromomethylbiphenyl-2-carboxylate **(2)** was added and the obtained mixture was stirred at room temperature (25-30°C) for 3 h. At this stage, the reaction was quenched with water (400 ml) and stirred for 30 min. The reaction mass was filtered. The resulting wet cake was slurried in water (200 ml) and stirred for 30 min. The reaction mass was filtered and the obtained solid was dried under vacuum at 60-65°C until constant weight. 18.3 g obtained (93% HPLC).

### 3.2. Oxalate salt formation (3)

18.3 g of the product obtained in the previous step was dissolved in 150 ml of ethyl alcohol and the resulting solution was stirred at reflux temperature. 4.56 g (0.0361 mol) of oxalic acid was added. The resulting mixture was refluxed (75-80°C) for 3 h. The reaction mass was cooled down to 10-15°C and stirred for 3 h. The reaction mass was filtered and the solid was washed with ethyl alcohol (10 ml). The resulting wet solid was refluxed with methyl ethyl ketone (140 ml) for 2 h. The reaction mass was cooled to 10-15 °C, stirred for 2 h and filtered. Washed the cake with methyl ethyl ketone (20 ml). The resulting oxalate salt 3 was dried under vacuum at 80-82°C until constant weight.

| | |
|---|---|
| *Yield:* | *16.2g* |
| *Yield (molar):* | *74.6%* |
| *HPLC:* | *99.7%* |
| *M.P.:* | *165-175°C* |

### Example 4. Preparation of crude Telmisartan

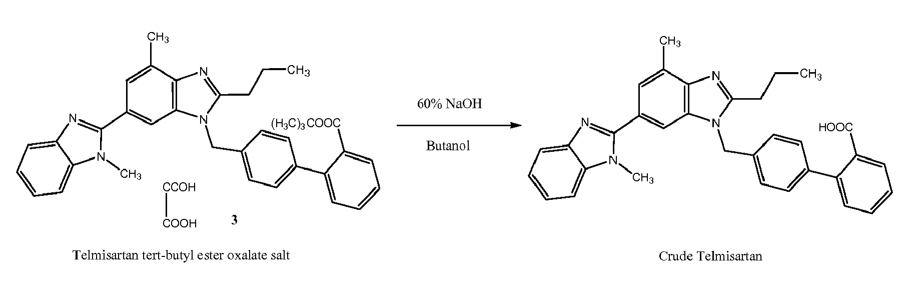

A solution of 14.5 g (0.36 mol) NaOH in 9.6 ml of water was prepared and stirred until obtaining a clear solution. To this solution, 128 ml of butanol was added. Afterwards, 16 g (0.024 mol) of compound **3,** obtained in the previous example, were added and the resulting mixture was heated at 115-120°C for 3-4 h. When the reaction was completed, cooled the reaction mass to 80°C and 240 ml of water was added. Separated the butanol layer and extracted the aqueous layer with butanol (48 ml). Combined the organic layers, washed with NaCl solution and the pH of the organic layer was adjusted to 4.5-5.0 with IPA HCl (22%). The reaction mass was stirred at room temperature (25°C-30°C) for 1 h. Afterwards, cooled the reaction mass to 10-15°C and stirred at this temperature for 4 h. The reaction mass was filtered. The generated cake was slurried in water (160 ml) for 30 min. Filtered the reactiom mass and washed with ethanol (40 ml) Finally, the obtained solid was dried under vacuum at 60-70°C until constant weight.

| | |
|---|---|
| *Yield:* | *11g* |
| *Yield (molar):* | *88.4%* |
| *HPLC:* | *99.74%* |

### Example 5. Purification of crude Telmisartan

11 g (0.0214 mol) of crude telmisartan obtained in the previous example was suspended in 110 ml of ethanol. After addition of 4 ml of ammonium hydroxide solution, the resulting mixture was stirred for 10 min. At this stage, 0.25 g of charcoal was added and the mixture was stirred at room temperature (25-30°C) for 30 min. Afterwards, the reaction mass was filtered through hyflow bed and the bed was washed with ethanol. pH of the obtained filtrate was adjusted to 4.5-5.0 with glacial acetic acid. The reaction mass was stirred for 1 h at room temperature (25-30°C), then cooled down to 10-15°C and stirred for 4 h. The reaction mass was filtered and the resulting cake was slurried in 110 ml of water. The resulting suspension was stirred for 30 min, filtered. The resulting cake was slurried with 110 ml of ethanol for1 h at room temperature. Filtered the mass and the solid was washed with ethanol. Finally, the product obtained was dried under vacuum at 60-65°C until constant weight.

| | |
|---|---|
| *Yield:* | *9 g* |
| *Yield (molar):* | *81.8%* |
| *HPLC:* | *99.83%* |

The obtained telmisartan is in the polymorphic form A, identified by its characteristic melting point an IR spectrum.

### Example 6. Preparation of other salts of telmisartan tert-butyl ester

### 6.1. Preparation of crude Telmisartan tert-butylester

10 g (0.0329 mol) of compound **1** was dissolved in DMSO (60 ml). The resulting solution was stirred at room temperature (25-30°C) until achieving a clear solution. 3.7 g (0.0329 mol) of Potassium *tert*-butoxide was added to the aforementioned solution and stirred at room temperature (25-30°C) for 30 min. Afterwards, the reaction mass was cooled down to 20°C and 11.44 g (0.0329 mol) of compound **2** was added and the obtained mixture was stirred at room temperature (25-30°C) for 3 h. At this stage, the reaction was quenched in water (400 ml) and extracted in MDC (100 ml). The organic layer was separated and the aqueous layer was extracted in MDC (50 ml). Both the organic layers were combined and washed twice with water (100 ml each). The MDC layer was dried over sodium sulphate and the MDC was distilled out completely. 18.5 g of crude telmisartan *tert*-butyl ester was obtained. HPLC purity is ∼90%.

Various salts of telmisartan *tert*-butyl ester were prepared from this crude telmisartan *tert*-butyl ester.

### 6.2. Salt of telmisartan tert-butyl ester with fumaric acid

5 g (0.00877 mol) of Telmisarta *tert*-Butyl ester, obtained as described in step 6.1., was treated with a solution of ethyl acetate (40 mL) and methanol (5 mL) and heated to reflux. Afterwards, a solution of fumaric acid (1.01 g, 0,0087 mol) dissolved in hot methanol (5 mL) was added and the mixture was refluxed for 2 h. The ethyl acetate was distilled partially (12 ml). Cooled down the reaction mass to room temperature (25-30°C) and stirred at this temperature for 1 h. Filtered the reaction mass and washed the cake with ethyl acetate. The resulting wet cake refluxed with acetone (50 ml) for 1 h. The reaction was cooled down to 10-15°C, stirred for 1 h, filtered and washed with acetone. The final product was dried at 50°C under vacuum until constant weight.

| | |
|---|---|
| *Yield:* | *4.3 g* |
| *Yield (molar):* | *72%* |
| *HPLC:* | *98.8%* |
| *M.P.:* | *175°C* |

### 6.3. Salt of telmisartan tert-butyl ester with succinic acid

5 g (0,0087 mol) of telmisartan *tert*-Butyl ester, obtained as described in step 6.1., was dissolved in methanol (25 ml) and 1,035 g (0,0087 mol) of succinic acid was added. The reaction mass was heated to reflux for 5 h. Afterwards, the solvent was distilled under vacuum. The resulting solid was suspended in hexane (30 ml) and refluxed for 30 min. Afterwards, the solvent was removed under vacuum. The resulting solid was again suspended in hexane (30 ml) and refluxed for 30 min. Cooled the reaction mass to room temperature (25-30°C), filtered and the generated solid was dried under vacuum until constant weight.

| | |
|---|---|
| *Yield:* | *5.3 g* |
| *Yield (molar):* | *88.5%* |
| *HPLC:* | *89.3%* |
| *M.P.:* | *113.2°C* |

### 6.4. Salt of telmisartan tert-butyl ester with citric acid

5 g (0,0087 mol) of telmisartan *tert*-butylester, obtained as described in step 6.1., was dissolved in methanol (25 ml) and 1,68 g (0,0087 mol) of citric acid was added. The reaction mass was heated to reflux for 5 h. Aferwards, the solvent was distilled under vacuum. The resulting solid was suspended in hexane (30 ml) and heated to reflux for 30 min. The obtained suspension was cooled down to room temperature (25-30°C), filtered and the generated solid was dried under vacuum at 50°C until constant weight.

| | |
|---|---|
| *Yield:* | *6.2 g* |
| *Yield (molar):* | *93.5%* |
| *HPLC:* | *90.57%* |
| *M.P.:* | *123. 3°C* |

### 6.5. Salt of telmisartan tert-butyl ester with maleic acid

5 g (0,0087 mol) of telmisartan *tert*-butilester, obtained as described in step 6.1. was dissolved in methanol (25 ml) and 1,01g (0,0087 mol) of maleic acid was added. The reaction mass was heated to reflux for 5 h. Afterwards, the solvent was removed under vacuum. Ethyl acetate (25 ml) was added to the resulting suspension and then it was stirred for 10 min. Afterwards, the solvent was removed under vacuum and hexane (25 ml) was added to the remaining solid. The suspension was heated to reflux temperature for 1 h. Then the solvent was removed completely under vacuum and hexane (25 ml) was added. The reaction mass was kept at 5-10°C for 24 h, filtered and the generated solid was dried under vacuum at 50°C until constant weight.

| | |
|---|---|
| *Yield:* | *4.2 g* |
| *Yield (molar):* | *70.37%* |
| *HPLC:* | *98.6%* |
| *M.P.:* | *149°C* |

### 6.6. Salt of telmisartan tert-butyl ester with tartaric acid

5 g (0,0087 mol) of telmisartan *tert*-butylester, obtained as described in step 6.1., was dissolved in methanol (25 ml) and 1,31 g (0,0087 mol) DL-tartaric acid was added. The reaction mass was heated under reflux for 5 h. Afterwards, the solvent was distilled under vacuum. 52 ml of acetone was added to the obtained residue and the suspension was stirred for 10 min. The solvent was distilled under vacuum completely. 30 ml of *tert*-Butylmethylether was added to the obtained residue and the suspension was heated to reflux for 1 h. Distill out the solvent completely under vacuum. Again, 30 ml of *tert*-Butylmethylether was added to the obtained residue and the suspension was heated to reflux for 1 h. Afterwards, the reaction mass was cooled down to 25-30°C, filtered and the resulted solid was dried under vacuum at 50°C until constant weight.

| | |
|---|---|
| *Yield:* | *4.3 g* |
| *Yield (molar):* | *68.64%* |
| *HPLC:* | *97.72%* |
| *M.P.:* | *174°C* |

### 6.7. Salt of telmisartan tert-butyl ester with trifluoroacetic acid

5 g (0,0087 mol) of telmisartan *tert*-butylester, obtained as described in step 6.1., was dissolved in 25 ml of ethyl acetate and 1,49 g (0,0137 mol) of trifluoro acetic acid was added. The reaction mass was heated under reflux for 5 h. Afterwards, the solvent was removed under vacuum. 25 ml of *tert*-Butylmethylether was added to the obtained residue and the suspension was heated to reflux for 4 h. At this stage, the solvent was removed under vacuum. Again, 30 ml of *tert*-Butylmethylether was added to the obtained residue and the suspension was heated to reflux for 30 min. Afterwards, the reaction mass was cooled down to 20°C, stirred for 2 h, filtered and the resulted solid was dried under vacuum at 50°C until constant weight.

| | |
|---|---|
| *Yield:* | *5.4 g* |
| *Yield (molar):* | *90%* |
| *HPLC:* | *98.6%* |
| *M.P.:* | *148.5°C* |

### 6.8. Salt of telmisartan tert-butyl ester with p-toluensulfonic acid

5 g (0,0087 mol) of telmisartan *tert*-butylester, obtained as described in step 6.1., was dissolved in 25 ml of ethyl acetate. 1,66 g (0,0087 mol) of *p*-toluenesulfonic acid was added. The reaction mass was heated under reflux temperature for 3 h. Afterwards, the solvent was removed under vacuum. 30 ml of *tert*-Butylmethylether was added to the obtained residue and the suspension was heated to reflux for 1 h. Cooled down the reaction mass to room temperature (25-30°C), filtered and the resulting solid was dried under vacuum until at 50°C constant weight.

| | |
|---|---|
| *Yield:* | *6.3 g* |
| *Yield (molar):* | *95.2%* |
| *HPLC:* | *92.5%* |
| *M.P.:* | *168.9°C* |

### 6.9. Salt of telmisartan tert-butyl ester with methanesulfonic acid

5 g (0,0087 mol) of telmisartan *tert*-butylester, obtained as described in step 6.1., was dissolved in 25 ml of ethyl acetate and 0,85 g (0,0087 mol) of methanesulfonic acid was added. The reaction mass was refluxed for 1 h. Solid precipitated out. The reaction mass was cooled down to room temperature (25-30°C) and the suspension was stirred for 30 min. Afterwards, the resulting suspension is filtered and the obtained solid was dried under vacuum at 50°C until constant weight.

| | |
|---|---|
| *Yield:* | *4 g* |
| *Yield (molar):* | *69%* |
| *HPLC:* | *98.34%* |
| *M.P.:* | *165°C* |

### Example 7. Preparation of crude telmisartan

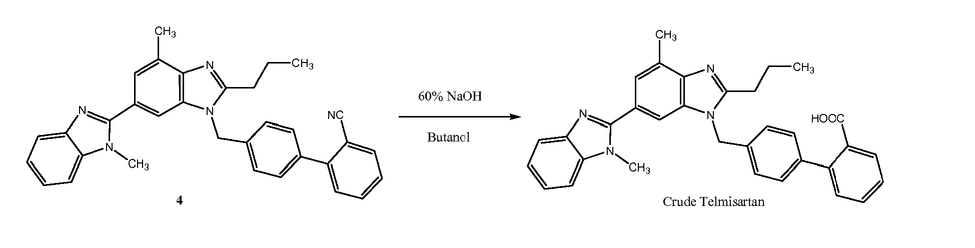

A solution of 47.3 g (1.18 mol) NaOH in 31.5 ml of water was prepared and stirred until a clear solution is formed. To this solution, 200 ml of butanol were added. Afterwards, 1 g (0.0029 mol) of tetrabutyl ammonium hydrogen sulphate was added and the temperature was increased to 115-120°C to get a clear solution. Then, 10 g (0.02 mol) of compound **4** was added and the temperature was maintained at 115-120°C for 22 h. After completion of reaction, cooled down the reaction mass to 50-55°C and 100 ml of water was added. Stirred the reaction mass for 30 min. Afterwards, the organic layer and the aqueous layer were separated and 100 ml of water was added to the organic layer and stirred during 30 min. Once separated, the organic and the aqueous layers, the two aqueous layers were combined and 20 ml of butanol was added. The mixture was stirred 30 min and then separated the layers. The organic layers were combined and the n-butanol was completely distilled out. 200 ml of water and 1 g of charcoal were added to the residue and stirred at 65°-70°C for 30 min. The reaction mass was hot filtered through hiflow. pH of the filtrate was adjusted to 4.5-5.0 with dilute hydrochloric acid at a temperature of 30°-35°C. The obtained mixture was stirred for 1 h, filtered and the solid was washed with 100 ml water. 160 ml of acetone were added and the resulting mixture was stirred for 1 h. Finally, the obtained wet cake was washed with acetone (10 ml) and dried under vacuum at 65-70 °C until constant weight.

| | |
|---|---|
| *Yield:* | *8.4 g* |
| *Yield (molar):* | *84%* |
| *HPLC:* | *98. 64%* |
| *Melting point:* | *262°-264°C* |

### Example 8. Purification of crude telmisartan

5 g (0.0097 mol) of crude telmisartan obtained in the previous example was suspended in 75 ml of ethyl alcohol. After addition of 0.33 ml of liquid ammonia, the resulting mixture was stirred and heated at 75-80°C. At this stage, 0.5 g of charcoal was added and the mixture was stirred at 75-80°C during 30 min. Afterwards, the suspension was filtered through hyflow bed and the filtrate was cooled at 25-30°C. pH of the obtained filtrate was adjusted to 4.5-5.0 with glacial acetic acid. The reaction mass was stirred during 1 h, filtered and the resulting cake was washed with 50 ml water and suck dried. The resulting cake was stirred in 75 ml of acetone for 30 min. Finally, the suspension was filtered and the product obtained was dried under vacuum at 60-65°C until constant weight.

| | |
|---|---|
| *Yield:* | *3.8 g* |
| *Yield (molar):* | *76%* |
| *HPLC:* | *99.82%* |

The obtained telmisartan is in the polymorphic form A, identified by its characteristic melting point and IR spectrum.

## Claims

1. A process for the preparation of telmisartan or salts thereof which comprises the hydrolysis of compounds of general formula I or salts thereof 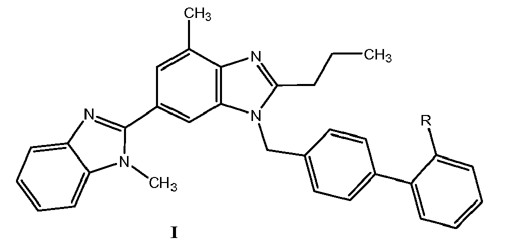 **characterized in that** the hydrolysis is carried out in a reaction medium comprising an aqueous basic solution and a polar protic solvent and **in that** R is selected from COOC(CH₃)₃ (Ia) and CN (Ib).

2. The process of claim 1 wherein the aqueous basic solution is a NaOH solution 60% (w/w) and the polar protic solvent is butanol.

3. The process of claim 1, wherein the reaction medium further comprises a phase transfer catalyst.

4. The process of claim 3, wherein the phase transfer catalyst is selected from tetrabutyl ammonium hydrogen sulfate, tetrabutyl ammonium bromide and 18-crown-6-ether, preferably, tetrabutyl ammonium hydrogen sulfate.

5. A salt of the compound of general formula I, wherein R is COOC(CH₃)₃ (Ia), and wherein HA is an organic acid.

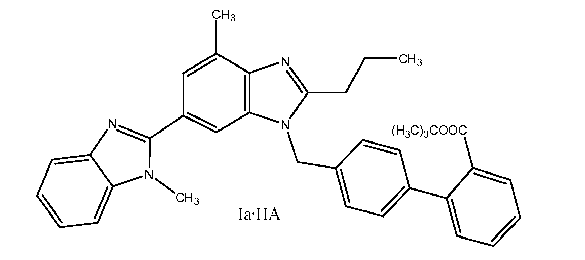

6. The salt according to claim 5, wherein HA is selected from fumaric acid, oxalic acid, succinic acid, citric acid, maleic acid, tartaric acid, trifluoro acetic acid, p-toluenesulfonic acid and methanesulfonic acid, preferably the oxalic acid.

7. A process for the preparation of telmisartan according to claim 1 wherein the hydrolysis step is carried out on the salt of the compound of formula Ia.HA, wherein HA is an organic acid, 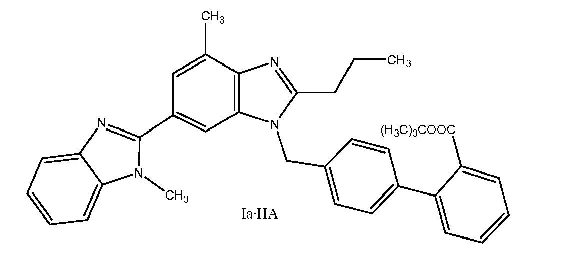 and further comprises the isolation of the obtained crude telmisartan and, optionally, the purification of crude telmisartan.

8. A process for the preparation of a salt of claim 5, which comprises:
a) Reacting 4-methyl-6-(1-methylbenzimidazol-2-yl)-2-n-propyl-1H-benzimidazole with *tert*-butyl-4'-(bromomethyl)-2-biphenyl-2-carboxylate in the presence of a base in an organic solvent
b) Adding an organic acid (HA)
c) Optionally, purifying the obtained salt

9. The process of claim 8 wherein in the step a) the base is selected from *tert-*butoxide, sodium methoxide, sodium ethoxide, potassium hydroxide and the like, more preferably potassium *tert*-butoxide

10. The process of claim 8 wherein in the step a) the organic solvent is selected from methanol, dimethylformamide, dimethylsulfoxide, isopropanol, ethyl acetate, acetonitrile, acetone, water and mixtures thereof, preferably, the solvent is dimethylsulfoxide.

11. The process of claim 8 wherein in the step b) the organic acid is selected from fumaric acid, oxalic acid, succinic acid, citric acid, maleic acid, tartaric acid, trifluoroacetic acid, p-toluenesulfonic acid and methanesulfonic acid, preferably, oxalic acid.

12. The process of claim 8 wherein in the step b) the solvent is selected from ethyl acetate, n-propyl acetate, methylene dichloride (MDC), isopropyl alcohol, methanol, ethanol and methyl ethyl ketone or mixtures thereof.

13. Use of salts of claim 5 for the preparation of telmisartan and salts thereof.
